# EUROPEAN PATENT SPECIFICATION

(11) **EP 0 672 645 B1**
(45) Date of publication and mention of the grant of the patent: **14.10.1998**
(21) Application number: 95100070.2
(22) Date of filing: 04.01.1995
(51) Int. Cl.: C07C 69/68, C07C 67/20

(54) **Process for preparing lactate**
Verfahren zur Herstellung von Milchsäureestern
Procédé de préparation de lactates

(30) Priority: 21.02.1994 JP 22385/94
(43) Date of publication of application: 20.09.1995
(73) Proprietor: MITSUBISHI GAS CHEMICAL COMPANY, INC., Chiyoda-ku, Tokyo (JP)
(72) Inventor: Abe, Takafumi, c/o Mitsubishi Gas Chem. Co.,Inc., Niigata-shi, Niigata-ken (JP); Gotoh, Toshiyuki, c/o Mitsubishi Gas Chem. Co.Inc., Niigata-shi, Niigata-ken (JP); Uchiyama, Takako, c/o Mitsubishi Gas Chem. Co.Inc., Niigata-shi, Niigata-ken (JP); Higuchi, Hirofumi, c/o Mitsubishi Gas Chem.Co.Inc., Niigata-shi, Niigata-ken (JP)
(74) Representative: Türk, Gille, Hrabal, Leifert

(56) References cited:
- EP-A- 0 342 458
- EP-A- 0 406 676
- EP-A- 0 487 853
- PATENT ABSTRACTS OF JAPAN vol. 7, no. 143 (C-172) (1288) 22 June 1983 & JP-A-58 055 444 (MITSUBISHI GAS KAGAKU K.K.)
- PATENT ABSTRACTS OF JAPAN vol. 14, no. 68 (C-0686) 8 February 1990 & JP-A-01 290 653 (MITSUBISHI GAS CHEM CO INC) 22 November 1989

## Description

### 1. Field of the Invention

The present invention relates to a process for preparing a lactate, and more specifically, it relates to a novel process for preparing a lactate from acetaldehyde and a formate which are starting materials. The lactates have been used in large quantities as coating materials, solvents for use in an electronic industry, materials for organic synthesis of medicines and the like, and materials for various polymers such as acrylic resins and biologically degradable polymers. Therefore, the lactates are industrially extremely important chemicals.

### 2. Description of the Related Art

An industrial method for preparing a lactate usually comprises synthesizing cyanohydrin from prussic acid and acetaldehyde as starting materials, hydrolyzing the same, and then esterifying.

As the other techniques for preparing lactic acid, there are known a method which comprises allowing dinitrogen tetroxide to act on a terminal olefin, followed by hydrolysis; a method which comprises reacting acetaldehyde with carbon monoxide and water in the presence of a noble metal catalyst or an acidic catalyst; and a method which comprises halogenating the α-position of a carboxylic acid, followed by hydrolysis. These methods are poor in yields, utilize the limited material sources, require troublesome operations of the reactions, separation and purification, and also require the expensive catalyst. For these reasons, the above-mentioned methods are ineligible for industrial methods for preparing lactic acid on a large scale. In fact, these methods have been used to specifically produce lactic acid and its derivatives only on a small scale.

The above-mentioned conventional method which comprises the synthesis of cyanohydrin from prussic acid and acetaldehyde, its hydrolysis and esterification has been widely utilized, because of the easy reaction and a high yield. In this method, however, a large amount of ammonium salts are formed as by-products, and the treatment of these by-products leads to the increase in a lactate manufacturing cost inconveniently.

EP-A-0 406 676 discloses a process for producing methacrylic acid from acetone and prussic acid via acetone cyanhydrine without undesirable by-products such as ammonium sulfate. This process includes a dehydration step of the intermediate compound α-hydroxyisobutyric acid amine.

EP-A-0 342 458 describes the production of carboxylic acid esters by reacting carboxylic acid amides and formic acid esters, or carboxylic acid amides, alcohols and carbon monoxide in the presence of metal alcoholates. The yields in this process calculated on the basis of "conversion x selectivity" are relatively low, i. e. between 54 and 72 % calculated from the examples disclosed therein.

### SUMMARY OF THE INVENTION

The present inventors have previously found a process for preparing methacrylic acid from acetone and methyl formate as starting materials via acetone cyanohydrin without forming the by-products of ammonium salts (Japanese Patent Application Laid-open No. 198152/1992 and U.S. Patent No. 5,225,594).

The present inventors have further researched, and as a result, it has been found that a lactate can efficiently be prepared by using acetaldehyde in place of acetone as a starting material in the above-mentioned process. The present invention has been completed on the basis of this knowledge.

That is to say, according to the present invention, there is provided a process for preparing a lactate represented by the general formula CH₃CH(OH)COOR (R is an alkyl group having 1 to 8 carbon atoms) which comprises (1) a step of preparing lactonitrile from prussic acid and acetaldehyde, (2) a step of hydrating lactonitrile obtained in the previous step to form lactamide, (3) a step of forming the lactate represented by the general formula CH₃CH(OH)COOR (R is as defined above) and formamide from lactamide obtained in the previous step and a formate represented by the general formula HCOOR (R is as defined above), and (4) a step of dehydrating formamide separated from the product obtained in the previous step to form prussic acid and recycling the same.

The process of the present invention can be achieved via the formation of lactonitrile, but according to this process, on the whole, the lactate can eventually be prepared from acetaldehyde and the formate as the starting materials. Hence, the process of the present invention is characterized by involving no formation of ammonium salts as by-products in contrast to a conventional technique.

### DESCRIPTION OF THE PREFERRED EMBODIMENTS

Next, a process of the present invention will be described in detail.

In the process of the present invention, acetaldehyde obtained by any method can be applied. In general, acetaldehyde has been utilized as a solvent or a material of various chemicals, and it has industrially been manufactured from ethylene in large quantities at a low cost by oxidation. In the present invention, acetaldehyde manufactured in this manner can usually be employed.

On the other hand, of formates for use in the process of the present invention, methyl formate obtained by any method can be used, but in general, there can be applied methyl formate manufactured on an industrial scale by a carbonylation method or a dehydrogenation method from methanol as a material, which has been produced in an extremely large amount at an extremely low cost. Furthermore, another formate, i.e., a formate (e.g., ethyl formate or propyl formate) represented by the general formula HCOOR' (wherein R' is an alkyl group having 2 to 8 carbon atoms) can easily be manufactured by ester interexchange between methyl formate and an alcohol represented by the general formula R'OH (R' is as defined above), or the like.

In the present invention, the manufacture of lactonitrile by the reaction of prussic acid and acetaldehyde has been carried out by a known technique, and it can easily be achieved at a low temperature of about 10°C in the presence of a basic catalyst such as an alkaline metal hydroxide, ammonia or an amine.

Lactamide in the present invention can be prepared by a catalytic reaction (a hydration reaction) of a mixture of lactonitrile and water in the presence of a catalyst. As this catalyst, applicable is a catalyst effective for the hydration reaction of a nitrile. A strong acid such as sulfuric acid can also be used, but the employment of a metallic catalyst or a metal oxide catalyst is preferable from the viewpoints of handling and economy. Concretely, manganese, copper, nickel, or its oxide is effective, and manganese is particularly preferable.

No particular restriction is put on a feed weight ratio of lactonitrile to water, and it can be suitably selected in compliance with given situations. Nevertheless, the feed weight ratio is in the proper range of 10:90 to 90:10. In this system, acetaldehyde which is the material of lactonitrile, an alcohol or a ketone such as ace-tone can also be allowed to coexist as a solvent.

In the case that manganese oxide is used as the catalyst, the reaction temperature is preferably in the range of 10 to 150°C, more preferably 20 to 100°C. The reaction time is preferably in the range of 0.3 to 6 hours, more preferably 0.5 to 3 hours. The reaction can be accomplished by either system of a batch system and a continuous system.

In the present invention, the production of a lactate and formamide by the reaction of lactamide and the formate can be carried out by heating a mixture of lactamide and the formate in the absence of a catalyst, but it is effective to do the reaction in the presence of a solvent and a catalyst. When the employment of methyl formate is acceptable as the formate, methyl formate may be replaced with methanol and carbon monoxide.

This reaction is an equilibrium reaction, and the yield of the lactate depends upon a feed molar ratio of lactamide to the formate. The feed molar ratio of formate/lactamide is preferably in the range of 1 to 10, more preferably 2 to 8.

The addition of the solvent enhances the solubility of lactamide which is solid, and increases the selectivity of the reaction. The most preferable solvent is an alcohol corresponding to the formate, and a feed molar ratio of the alcohol to lactamide is preferably in the range of 1 to 10, more preferably 2 to 8.

As the catalyst for use in the above-mentioned reaction, i.e., the reaction of lactamide and the formate, alcoholates of alkaline metals, oxides of alkaline earth metals and strongly basic ion exchange resins are extremely excellent. The alcoholates of the alkaline metals can be synthesized from metals such as lithium, sodium and potassium and lower alcohols. Typical examples of the alcoholates of the alkaline metals include methylates, ethylates and butylates of sodium and potassium. Furthermore, examples of the oxides of the alkaline earth metals include magnesium oxide, calcium oxide and barium oxide.

As reaction conditions in the case that the alcoholate of the alkaline metal, the oxide of the alkaline earth metal or the strongly basic ion exchange resin is used as the catalyst, the reaction temperature is in the range of 20 to 80°C, the reaction time is in the range of 0.5 to 6 hours, and the amount of the catalyst to be used is suitably in the range of 0.001 to 0.30 mol per mol of lactamide.

The reaction product in this process can be separated and collected by an operation such as distillation, and an unreacted material is returned to a material system.

Formamide which is produced together with the desired lactate is subjected to a dehydration reaction to form prussic acid. The thus formed prussic acid is recovered and returned to the cyanohydrin preparation step and then reused.

According to the present invention, each step proceeds in a very high yield, and the lactate can eventually be prepared in a high yield from acetaldehyde and the formate as starting materials. In addition, the formation of inconvenient by-products such as ammonium salts in a Conventional method does not take place at all, and therefore it is fair to say that the present invention has an industrially extremely high value.

Next, the present invention will be described in more detail with reference to examples, but the scope of the present invention should not be limited to these examples.

### Example 1 (Synthesis of methyl lactate)

### Step (1): (Synthesis of lactonitrile from prussic acid and acetaldehyde)

In a 500-ml flask equipped with a stirrer, a thermometer, a reflux condenser and a dropping funnel were placed 88.1 g of acetaldehyde and 1 ml of a 1N aqueous sodium hydroxide solution, and 59.4 g of prussic acid was added dropwise thereto while the temperature in the flask was maintained at 10°C. After the addition of prussic acid, the solution was maintained at 20°C for 2 hours to complete the reaction. Next, 50% sulfuric acid was added thereto, thereby adjusting the pH of the produced solution to 3.

The flask was connected to a system under reduced pressure, and unreacted prussic acid was removed from the system, so that 142 g of lactonitrile was obtained as a residue. In the prussic acid fraction removed from the system, any lactonitrile was not detected.

The purity of the thus obtained lactonitrile was 98.8%, and the yield of lactonitrile based on acetaldehyde was 98.7%.

### Step (2): (Synthesis of lactamide by hydration of lactonitrile)

### Preparation of catalyst:

In a 1-liter flask equipped with a stirrer, a reflux condenser and a thermometer were placed 63.2 g of potassium permanganate and 500 g of water, followed by heating to 70°C and stirring. Next, 240 g of an aqueous solution containing 96.2 g of dissolved manganese sulfate and 40 g of 15% sulfuric acid were added to the solution, and reaction was then carried out at 70°C for 3 hours.

After the contents were cooled, the resulting precipitate was collected by suction filtration, and then washed with 2.4 ℓ of water. Next, the precipitate cake was dried at 60°C one whole day and night to obtain 74 g of active manganese dioxide. The thus obtained manganese dioxide would be used as a catalyst.

### Hydration reaction

In a 1-liter flask equipped with a stirrer, a reflux condenser and a thermometer were placed 121 g of lactonitrile obtained in the above-mentioned step (1), 350 g of water and 60 g of manganese dioxide in turn, and the resulting mixture was then heated and stirred at 60°C for 5 hours to carry out reaction.

After the produced solution was cooled on ice, the catalyst was separated by suction filtration. The resulting filtrate was subjected to gas chromatography analysis, and as a result, it was apparent that the conversion of lactonitrile was 99.5% and the yield of lactamide was 97.5%.

This filtrate was concentrated to dryness under reduced pressure, thereby obtaining 148 g of lactamide having a purity of 99.5% or more as a main component.

### Step (3): (Synthesis of methyl lactate and formamide from lactamide and methyl formate)

In a 1-liter stainless steel autoclave equipped with a stirrer were placed 44.5 g of lactamide obtained in the step (2), 180 g of methyl formate, 96 g of methanol and 1.1 g of sodium methylate, and they were then heated and stirred at 60°C for 2 hours to carry out reaction.

After the cooling of the product, gas chromatography analysis was carried out. As a result, it was apparent that the conversion of lactamide was 86.1%, the selectivity for methyl lactate and the selectivity for formamide based on lactamide was 99.8% and 98.4%, respectively.

Sodium methylate in the produced solution was neutralized with sulfuric acid, and the solution was then distilled in a usual manner to recover methyl formate and methanol and to simultaneously obtain 40 g of methyl lactate having a purity of 99% or more and 14 g of formamide having a purity of 99%. The recovery of these substances inclusive of an intermediate fraction was quantitative.

### Step (4): (Preparation of prussic acid by dehydration of formamide)

### Preparation of catalyst:

0.88 g of sodium carbonate dissolved in 30 g of water was added to 51.5 g of manganese carbonate, followed by kneading for 1 hour. Afterward, the mixture was dried at 110°C for 15 hours, calcined in a 10% hydrogen-nitrogen gas stream at 450°C for 5 hours, and then ground to obtain 30 g of a catalyst having a uniform size of 10 to 20 mesh.

### Reaction:

A quartz reaction tube having a size of 10 mm (internal diameter) × 300 m (length) and equipped with a thermometer sheath was filled with 3.0 g of manganese oxide obtained in the above-mentioned manner, and it was then heated so that the temperature of the lower portion of the resulting catalyst layer might be maintained at 400°C. Furthermore, the reaction tube was filled with quartz Raschig rings having a size of 3 mm (diameter) × 3 mm (length) as thick as 15 cm on the catalyst layer, and it was then heated up to 100 to 400°C to form a formamide evaporating section.

While the pressure in the reaction tube was maintained at a vacuum degree of 100 mmHg, formamide obtained in the above-mentioned step (3) and air were introduced into the system through the top of the reaction tube at feed rates of 10 g/hour and 240 ml/hour, respectively.

Five hours after the start of the reaction, the resulting reaction gas was sampled for 1 hour. Prussic acid collected by allowing water and an aqueous NaOH solution to absorb the same was determined by a silver nitrate titration. In addition, ammonia dissolved in water was determined by ion chromatography, and unreacted formamide was done by gas chromatography.

As a result, it was apparent that the conversion of formamide was 99.5%, the selectivity for prussic acid was 95.2%, and the yield of ammonia was 4.3%.

### Example 2

Reactions were carried out by the same procedure as in Example 1 except that methyl formate as a starting material was replaced with ethyl formate. As a result, it was apparent that the conversion of lactamide was 86.1%, the selectivity for ethyl lactate and the selectivity for formamide based on lactamide was 99.8% and 98.4%, respectively.

### Example 3

The same procedure as in Example 1 was carried out except that in the step (3) of Example 1, 180 g of methyl formate and 96 g of methanol were replaced with 200 g of methanol, and carbon monoxide was introduced at 40 atm, followed by heating and stirring to perform reaction. When the temperature in an autoclave had reached 60°C, carbon monoxide was fed so that reaction pressure might be maintained at 40 atm, and the reaction was continued for 3 hours.

Afterward, the temperature in the autoclave was cooled to 10°C, and the internal pressure was slowly lowered to atmospheric pressure. Afterward, the resulting product was taken out, and then subjected to gas chromatography. As a result, it was apparent that the conversion of lactamide was 81.7%, the selectivity for methyl lactate and the selectivity for formamide based on lactamide was 95.9% and 94.8%, respectively.

## Claims

1. A process for preparing a lactate represented by the general formula CH₃CH(OH)COOR (R is an alkyl group having 1 to 8 carbon atoms) which comprises (1) a step of preparing lactonitrile from prussic acid and acetaldehyde, (2) a step of hydrating lactonitrile obtained in the previous step to form lactamide, (3) a step of forming the lactate represented by the general formula CH₃CH(OH)COOR (R is as defined above) and formamide from lactamide obtained in the previous step and a formate represented by the general formula HCOOR (R is as defined above), and (4) a step of dehydrating formamide separated from the product obtained in the previous step to form prussic acid and recycling the same.

2. The process for preparing a lactate according to Claim 1 wherein the formate used in the step (3) is methyl formate.

3. The process for preparing a lactate according to Claim 2 wherein in the step (3), methanol and carbon monoxide are used in place of methyl formate.

4. The process for preparing a lactate according to Claim 1 wherein the step (1) is carried out in the presence of a basic catalyst.

5. The process for preparing a lactate according to Claim 1 wherein the step (2) is carried out in the presence of a catalyst comprising manganese, copper, nickel or their oxides.

6. The process for preparing a lactate according to Claim 1 wherein the step (3) is carried out in the presence of a catalyst comprising an alcoholate of an alkaline metal, an oxide of an alkaline earth metal or a strongly basic ion exchange resin.

7. The process for preparing a lactate according to Claim 1 wherein the step (1) is carried out in the presence of a basic catalyst, the step (2) is done in the presence of a catalyst comprising manganese, copper, nickel or their oxides and the step (3) is done in the presence of a catalyst comprising an alcoholate of an alkaline metal, an oxide of an alkaline earth metal or a strongly basic ion exchange resin.

## Patentansprüche

1. Verfahren zur Herstellung eines Lactats der allgemeinen Formel CH₃CH(OH)COOR (R ist eine Alkygruppe mit 1 - 8 Kohlenstoffatomen), welches umfaßt
(1) den Schritt der Herstellung von Lactonitril aus Blausäure und Acetaldehyd
(2) den Schritt der Hydratisierung von Lactonitril, erhalten im vorhergehenden Schritt, unter Bildung von Lactamid,
(3) den Schritt der Bildung des Lactats der allgemeinen Formel CH₃CH(OH)COOR (R ist wie oben definiert) und Formamid aus Lactamid, erhalten im vorhergehenden Schritt, und ein Formiat der allgemeinen Formel HCOOR (R = ist wie oben definiert) und
(4) den Schritt der Dehydratisierung von Formamid, abgetrennt vom im vorhergehenden Schritt erhaltenen Produkt, zur Bildung von Blausäure und Rückführung derselben.

2. Das Verfahren zur Herstellung von Lactat gemäß Anspruch 1, wobei das im Schritt (3) verwendete Formiat Methylformiat ist.

3. Das Verfahren zur Herstellung eines Lactats gemäß Anspruch 2, worin in Schritt (3) anstelle von Methylformiat Methanol und Kohlenmonoxid verwendet werden.

4. Das Verfahren zur Herstellung von Lactat gemäß Anspruch 1, wobei Schritt (1) in Gegenwart eines basischen Katalysators durchgeführt wird.

5. Das Verfahren zur Herstellung eines Lactats gemäß Anspruch 1, wobei Schritt (2) in Gegenwart eines Katalysators umfassend Mangan, Kupfer, Nickel oder deren Oxide durchgeführt wird.

6. Das Verfahren zur Herstellung eines Lactats gemäß Anspruch 1, wobei Schritt (3) in Gegenwart eines Katalysators, umfassend ein Alkalimetallalkoholat, ein Erdalkalimetalloxid oder ein stark basisches Ionenaustauscherharz, durchgeführt wird.

7. Das Verfahren zur Herstellung eines Lactats gemäß Anspruch 1, worin Schritt (1) in Gegenwart eines basischen Katalysators durchgeführt wird, Schritt (2) in Gegenwart eines Katalysators, umfassend Mangan, Kupfer, Nickel oder deren Oxide durchgeführt wird und Schritt (3) in Gegenwart eines Katalysators, umfassend ein Alkalimetallalkoholat, ein Erdalkalimetalloxid oder ein stark basisches Ionenaustauscherharz, durchgeführt wird.

## Revendications

1. Procédé de préparation d'un lactate représenté par la formule générale :
CH₃CH(OH)COOR (R étant un groupe alkyle ayant 1 à 8 atomes de carbone)
qui comprend (1) un stade de préparation de lactonitrile à partir d'acide prussique et d'acétaldéhyde, (2) un stade d'hydratation de lactonitrile obtenu au stade antérieur pour former un lactamide, (3) un stade de formation de lactate représenté par la formule générale :
CH₃CH(OH)COOR (R étant comme défini ci-dessus) et de formamide à partir du lactamide obtenu au stade antérieur et un formiate représenté par la formule générale:
HCOOR (R étant comme défini ci-dessus) et (4) un stade de déshydratation du formamide Séparé du produit obtenu au stade antérieur pour former l'acide prussique et son recyclage.

2. Procédé de préparation d'un lactate selon la revendication 1, dans lequel le formiate utilisé au stade (3) est un formiate de méthyle.

3. Procédé de préparation d'un lactate selon la revendication 2, dans lequel au stade (3), on utilise du méthanol et de l'oxyde de carbone à la place du formiate de méthyle.

4. Procédé de préparation d'un lactate selon la revendication l, dans lequel le stade (1) est effectué en présence d'un catalyseur basique.

5. Procédé de préparation d'un lactate selon la revendication 1, dans lequel le stade (2) est effectué en présence d'un catalyseur comprenant du manganèse, du cuivre, du nickel ou leurs oxydes.

6. Procédé de préparation d'un lactate selon la revendication 1, dans lequel le stade (3) est effectué en présence d'un catalyseur comprenant un alcoolate d'un métal alcalin, un oxyde d'un métal alcalino-terreux ou une résine à échange ionique fortement basique.

7. Procédé de préparation d'un lactate selon la revendication 1, dans lequel on effectue le stade (1) en présence d'un catalyseur basique, on effectue le stade (2) en présence d'un catalyseur comprenant du manganèse, du cuivre, du nickel ou leurs oxydes et on effectue le stade (3) en présence d'un catalyseur comprenant un alcoolate d'un métal alcalin, un oxyde d'un métal alcalino-terreux ou une résine à échange ionique fortement basique.
